# EUROPEAN PATENT APPLICATION

(11) **EP 4 497 374 A1**
(43) Date of publication of application: **29.01.2025**
(21) Application number: 23775251.4
(22) Date of filing: 21.03.2023
(51) Int. Cl.: A61B 5/00, A61C 19/04, G06T 17/20, G06T 7/30

(54) **METHOD OF PRODUCING 3-DIMENSIONAL FACE SCAN DATA**

(30) Priority: 22.03.2022 KR 20220035664
(71) Applicant: Ray Co., Ltd., Seongnam-si, Gyeonggi-do 13494 (KR)
(72) Inventor: LEE, Sang Chul, Seongnam-si Gyeonggi-do 13558 (KR); SHIN, Seung Hoon, Seongnam-si Gyeonggi-do 13429 (KR)
(74) Representative: Studio Torta S.p.A.
(86) International application number: PCT/KR2023/003695
(87) International publication number: WO 2023/182755

(57) **Abstract**

A method of producing three-dimensional face scan data according to the present disclosure includes the steps of: acquiring a face image obtained by photographing a face of a patient; generating 3D data from the face image; acquiring a tooth area image obtained by photographing a tooth area of the patient; generating tooth area 3D data from the tooth area image; extracting a tooth area contour for the face image or the face 3D data; separating and acquiring tooth area contour 3D data corresponding to the tooth area contour from the tooth area 3D data; and aligning and inserting the tooth area contour 3D data to the face 3D data.

## Description

### BACKGROUND

### TECHNICAL FIELD

The present disclosure generally relates to a technology for generating three-dimensional (3D) face scan data of a person.

In particular, the present disclosure relates to a technology for generating 3D face scan data, which photographs respective face and tooth areas with a structured light pattern projected thereon to individually acquire 3D data for the face and tooth areas, extract a tooth zone contour using face landmarks, and align and insert tooth zone contour 3D data, which is obtained by separating the extracted tooth zone contour from the tooth area 3D data, into the face 3D data.

### DESCRIPTION OF RELATED ART

For cosmetic dental treatment, obtaining 3D face scan data by scanning a face of a patient in three dimensions is necessary. For example, for orthodontic treatment planning, 3D face scan data of the face of the patient is crucial for visualizing the resulting facial appearance throughout the orthodontic treatment process.

To this end, a specialized device such as Bellus3D configured to photograph the patient's whole face at once in three dimensions is used. However, this device is expensive and unsuitable for dental use because photographing the whole face at once compromises the precision of 3D data of the tooth area.

On the other hand, a device such as Face Hunter that scans the face of the patient may be tried. However, this device also photographs the face in a single scan such that data precision of the tooth area data is compromised.
(PTL 0001) Korean Patent Publication No. 10-2020-0014645 (2020.02.11)
   "Method for fabricating customized dental implant using oral cavity scanning"
(PTL 0002) Korean Patent Publication No. 10-2022-0023914 (2022.03.03) "Dental scan body"
(PTL 0003) Korean Registered Patent No. 10-1176770 (2012.08.17) "Dental 3-Dimensional Scanner and Method of Scanning by Using the Same"
(PTL 0004) Korean Registered Patent No. 10-2334519 (2021.11.30) "Simplified automatic face landmark detection method from dental 3D scan data"
(PTL 0005) Korean Registered Patent No. 10-1744079 (2017.05.31) "The face model generation method for the dental procedure simulation"

### SUMMARY

In order to solve one or more problems (e.g., the problems described above and/or other problems not explicitly described herein), the present disclosure provides a technology for generating 3D face scan data of a person.

In particular, the present disclosure provides a technology for generating 3D face scan data, which photographs respective face and tooth areas with a structured light pattern projected thereon to individually acquire 3D data for the face and tooth areas, extract a tooth zone contour using face landmarks, and align and insert tooth zone contour 3D data, which is obtained by separating the extracted tooth zone contour from the tooth area 3D data, into the face 3D data.

On the other hand, the issues to be addressed by the present disclosure are not limited to the above, and other issues not explicitly described herein may be understood from the description.

A method for generating 3D face scan data may include acquiring a face image obtained by photographing a face of a patient, generating face 3D data from the face image acquiring a tooth area image obtained by photographing a tooth area of the patient, generating tooth area 3D data from the tooth area image, extracting a tooth zone contour for the face image or the face 3D data, separating and acquiring tooth zone contour 3D data corresponding to the tooth zone contour from the tooth area 3D data, and aligning and inserting the tooth zone contour 3D data into the face 3D data.

In the method for generating 3D face scan data, the acquiring the tooth area image obtained by photographing the tooth area of the patient may include acquiring a plurality of partial tooth area images obtained by photographing the tooth area of the patient from a plurality of angles, and the generating tooth area 3D data from the tooth area image may include generating a plurality of partial tooth area 3D data from the plurality of partial tooth area images, and generating tooth area 3D data for the whole tooth area by aligning the plurality of partial tooth area 3D data.

In the method for generating 3D face scan data, the aligning and inserting the tooth zone contour 3D data into the face 3D data may include removing data of the tooth zone contour portion from the face 3D data, and aligning and inserting the tooth zone contour 3D data into the tooth zone contour portion of the face 3D data.

In the method for generating 3D face scan data, the method may further include generating auxiliary area 3D data for a preset area of a face and aligning and inserting the auxiliary area 3D data into the face 3D data.

In the method for generating 3D face scan data, the face image, the face 3D data, the tooth area image, and the tooth area 3D data may be acquired while a structured light pattern is projected onto the face or the tooth area of the patient.

In the method for generating 3D face scan data, the extracting the tooth zone contour for the face image or the face 3D data may include recognizing face landmarks from the face image or the face 3D data, and extracting a tooth zone contour for the face image or the face 3D data on the basis of the face landmarks.

A computer program is provided, which may be stored in a non-transitory computer recordable storage medium to execute the method for generating 3D face scan data described above on a computer.

According to the present disclosure, 3D face scan data including high-resolution dental data can be generated through a relatively low-cost device, which has the advantage of effectively assisting in several fields (e.g., cosmetic dental treatment).

### BRIEF DESCRIPTION OF DRAWINGS

The above and other objects, features and advantages of the present disclosure will become more apparent to those of ordinary skill in the art by describing in detail exemplary embodiments thereof with reference to the accompanying drawings, in which:
FIG. 1 illustrates a 3D face scan system;
FIG. 2 is a flowchart provided to explain a method for generating a 3D face scan data;
FIG. 3 illustrates an image, photographed with a camera, of face and tooth areas with a structured light pattern projected thereon;
FIG. 4 is a view conceptually illustrating 3D data of the face and tooth areas;
FIG. 5 illustrates the concept of aligning 3D data of the face and tooth areas;
FIG. 6 illustrates the concept of extracting face landmarks and a tooth zone contour from a face image;
FIG. 7 illustrates the concept of separating and acquiring tooth zone contour 3D data from tooth area 3D data;
FIG. 8 illustrates the concept of removing a tooth zone contour portion from face 3D data and aligning and inserting tooth zone contour 3D data;
FIG. 9 illustrates a 3D face scan system; and
FIG. 10 is a flowchart illustrating a method for generating 3D face scan data.

### DETAILED DESCRIPTION

Hereinafter, example details for the practice of the present disclosure will be described in detail with reference to the accompanying drawings.

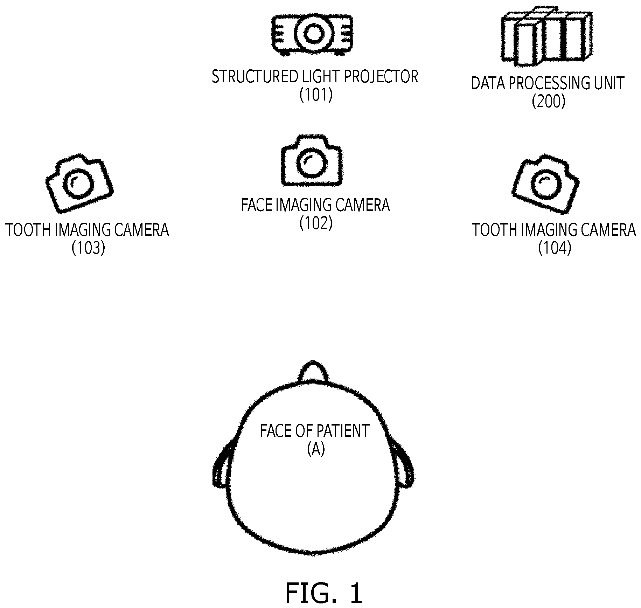

FIG. 1 illustrates a 3D face scan system.

Referring to FIG. 1, the 3D face scan system includes a structured light projector 101, a face imaging camera 102, tooth imaging cameras 103 and 104, and a data processing unit 200. These components may be implemented as separate devices, or some or all of the components may be integrated into a single unit.

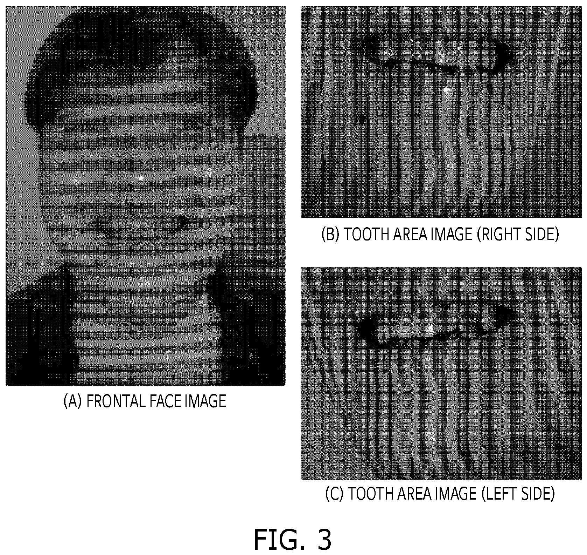

First, the structured light projector 101 is a device for generating a structured light pattern and projecting the structured light pattern onto a face A of a patient, as illustrated in FIG. 3. Meanwhile, other methods such as using a stereo camera instead of structured light, or using time of flight, etc. may be employed.

The face imaging camera 102 is a device for photographing the face A of the patient to acquire a face image such as that illustrated in FIG. 3A. Photographing the face A of the patient may be performed while the structured light pattern is projected onto the face A of the patient. In addition, photographing the face A of the patient may be performed from at least one of the positions on the front, right and left sides of, and below and above the face of the patient, but is not limited thereto.

The tooth imaging cameras 103 and 104 are devices for photographing the tooth area of the face A of the patient to acquire tooth area images such as those illustrated in FIGS. 3A, 3B, and 3C. At this time, the tooth area of the face of the patient may be photographed while the structured light pattern is projected onto the face A of the patient.

The face imaging camera 102 for photographing a face and the tooth imaging cameras 103 and 104 for exclusively photographing tooth area may be configured as separate devices. Only one of the tooth imaging cameras 103 and 104 may be provided, although it is preferable that a plurality of dental cameras are provided to photograph the patient's tooth area from multiple angles and acquire a plurality of tooth area images. FIG. 1 illustrates that two tooth imaging cameras 103 and 104 are provided. In this case, some areas may be visible from all of the plurality of cameras 103 and 104, and other areas may be visible only from part of the cameras 103 or 104, in which case the accuracy of the 3D data may be increased by image registration and synthesis.

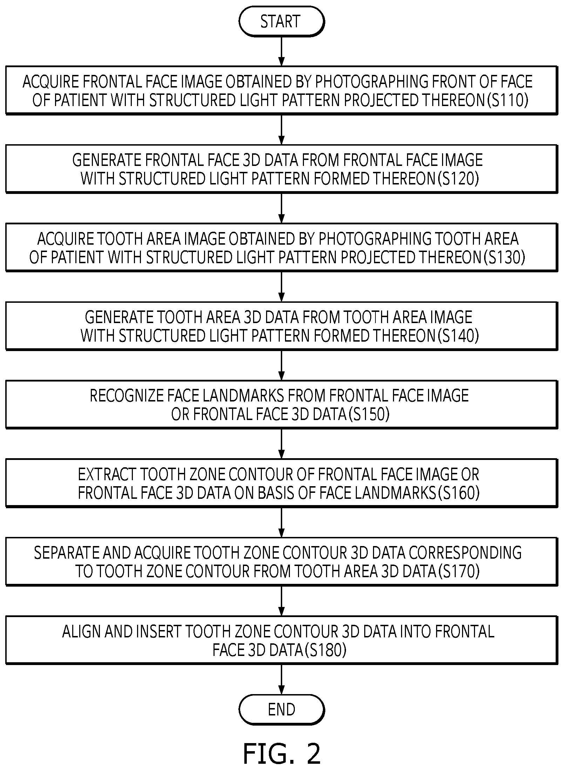

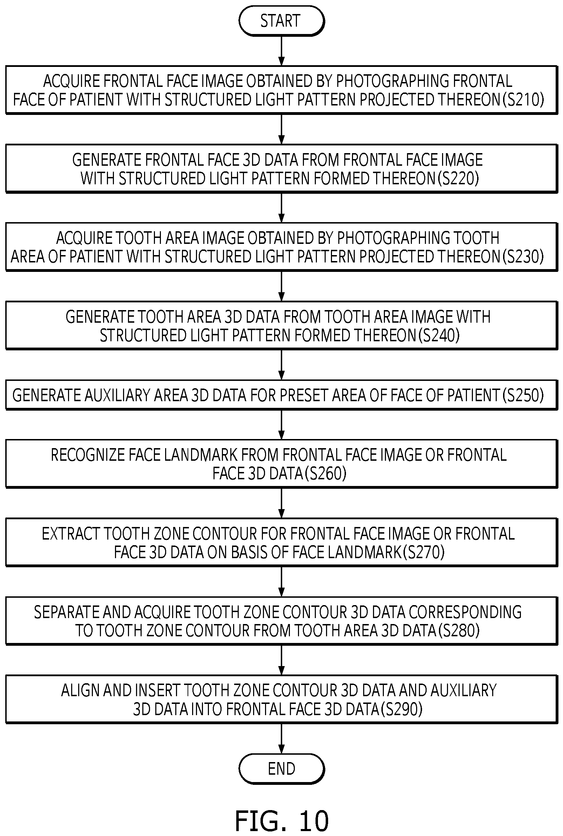

The data processing unit 200 is a device that receives the face image acquired by the face imaging camera 102 and the tooth area image acquired by the tooth imaging cameras 103 and 104, and generates 3D face scan data through a data processing process according to FIG. 2 or FIG. 10.

FIG. 2 is a flowing chart illustrating a method for generating 3D face scan data.

Operations S110 and S120: The face image such as that illustrated in FIG. 3A is acquired by photographing, through the face imaging camera 102, the face A of the patient while a preset structured light pattern is projected onto the face A of the patient by the structured light projector 101.

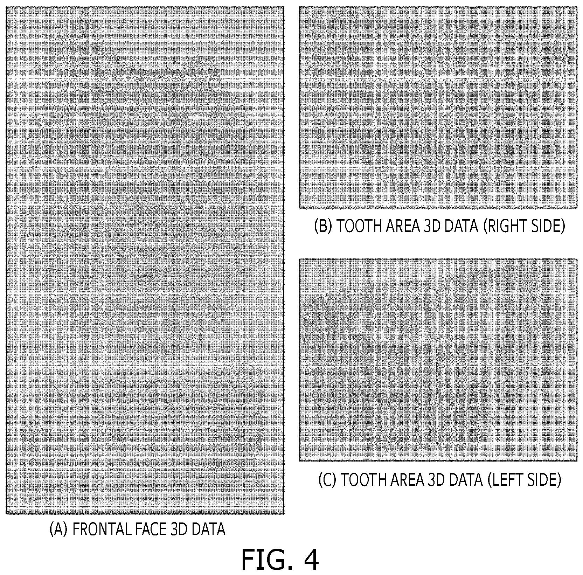

The face 3D data such as that illustrated in FIG. 4A is generated from the face image with the structured light pattern formed thereon. It is possible to generate the 3D data, because the 3D shape of the face can be identified with the structured light pattern. Since the technology for acquiring 3D data from an image of an object with a structured light pattern projected thereon is already known, detailed description thereof will be omitted.

Operations S130 and S140: Further, tooth area images such as those illustrated in FIGS. 3A, 3B, and 3C are acquired by photographing, through the tooth imaging cameras 103 and 104, the tooth area of a person while the preset structured light pattern is projected onto the face A of the same person by the structured light projector 101. At this time, the structured light pattern for the face image and the structured light pattern for the tooth area image may be the same or may be different.

Tooth area 3D data such as those illustrated in FIGS. 4A, 4B, and 4C is generated from the tooth area images with the structured light pattern formed thereon. Comparison between the tooth area images illustrates that the tooth area images of FIGS. 3B and 3C have much higher resolution than the tooth area image included in the face image of FIG. 3A. A natural consequence is that the tooth area 3D data of FIGS. 4B and 4C are much more detailed than the tooth zoon 3D data included in the face 3D data of FIG. 4A.

As described in FIG. 1, it is preferable that a plurality of tooth imaging cameras 103 and 104 are provided so as to acquire a plurality of partial tooth area images by photographing the tooth area from a plurality of angles in S130. In this case, the operation at S140 may include generating a plurality of partial tooth area 3D data such as those illustrated in FIGS. 4B and 4C from the plurality of partial tooth area images with the structured light pattern formed thereon, such as those illustrated in FIGS. 3B and 3C, and generating the tooth area 3D data for the whole tooth area of the patient by aligning the plurality of partial tooth area 3D data.

With some areas commonly visible from all of the plurality of tooth imaging cameras 103 and 104 and other areas visible only from any individual tooth imaging camera 103 or 104, it is possible to obtain substantially precise and detailed high-resolution 3D data of the tooth area of the patient by aligning and synthesizing these areas.

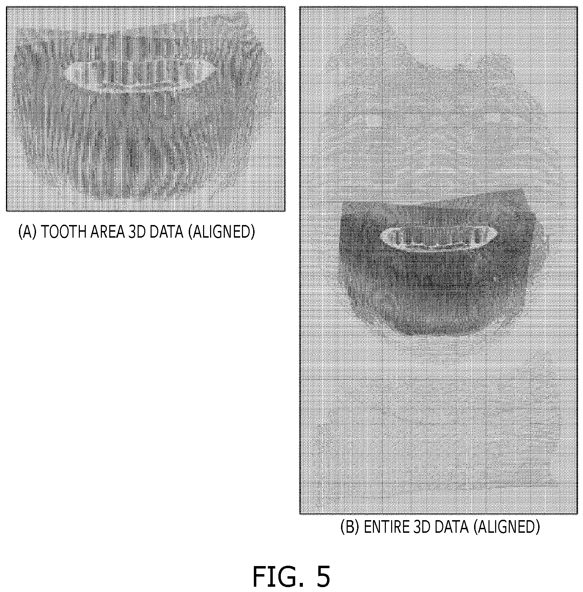

FIG. 5 illustrates the concept of aligning face and tooth area 3D data. FIG. 5A illustrates an example of tooth area 3D data generated by aligning two tooth area 3D data of FIGS. 4B and 4C, and FIG. 5B illustrates an example of aligning this tooth area 3D data with face 3D data.

Operations S150 and S160: Face landmarks of the face are recognized and a tooth zone contour of the face is extracted on the basis of the face landmarks. This process may be performed on the face image such as that illustrated in FIG. 3A or may be performed from the face 3D data such as that illustrated in FIG. 4A.

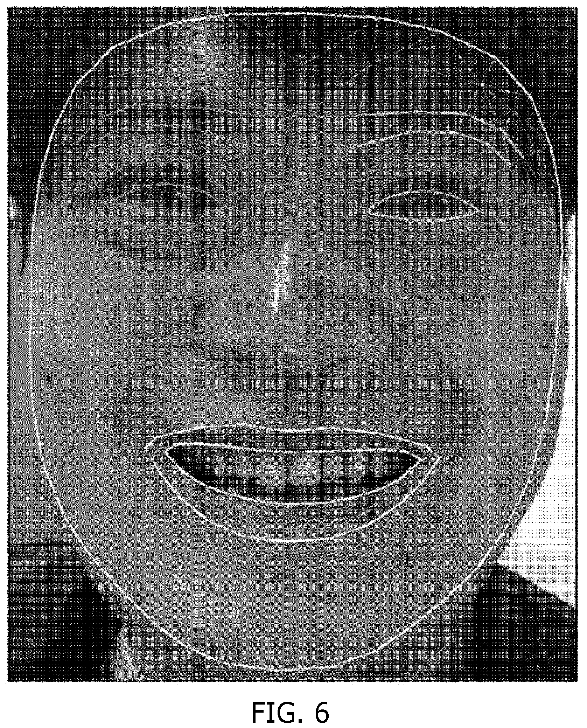

FIG. 6 illustrates the concept of extracting face landmarks and tooth zone contour from the face image such as that illustrated in FIG. 3A. Since the concept of the face landmarks and the technology of extracting the face landmarks from the face image are already known, their detailed description will be omitted. For example, a Google search for the keyword "face landmark detection" will yield a variety of techniques. The tooth zone contour corresponds to the boundary of the tooth area on the face, and the lip boundary is used as the tooth zone contour in FIG. 6.

On the other hand, using the face landmarks to extract the tooth area contour is an example. Accordingly, other methods may be used such as using AI-based object recognition algorithms, but aspects are not limited thereto.

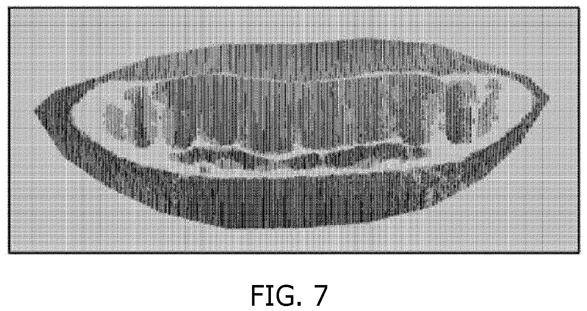

Operation S170: The tooth zone contour 3D data corresponding to the tooth zone contour extracted in S160 is separated and acquired from the tooth area 3D data (e.g., the tooth area 3D data of FIG. 5A) generated previously in S140. FIG. 7 illustrates the concept of separating and acquiring the tooth zone contour 3D data along the tooth zone contour from the tooth area 3D data.

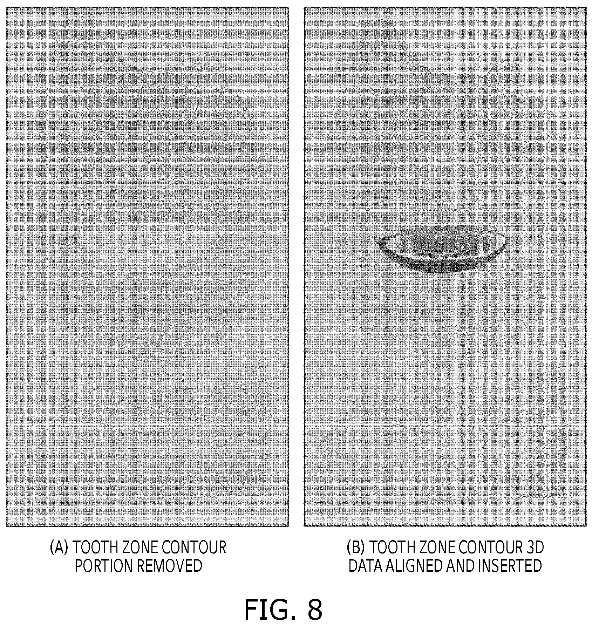

Operation S180: 3D face scan data is acquired by aligning and inserting the tooth zone contour 3D data separated and acquired in S170 into the face 3D data acquired in S120. FIG. 8 illustrates the concept of aligning and inserting the tooth zone contour 3D data into the face 3D data. In FIG. 8B, the tooth zone contour 3D data of the tooth zone contour is more detailed high-solution 3D data compared to the face 3D data that covers the whole face area.

Although the tooth zone contour 3D data may be directly augmented to the face 3D data, the data of the tooth zone contour portion may be removed from face 3D data as illustrated in FIG. 8A, before the tooth zone contour 3D data may be aligned and inserted into the tooth zone contour portion of the face 3D data as illustrated in FIG. 8B.

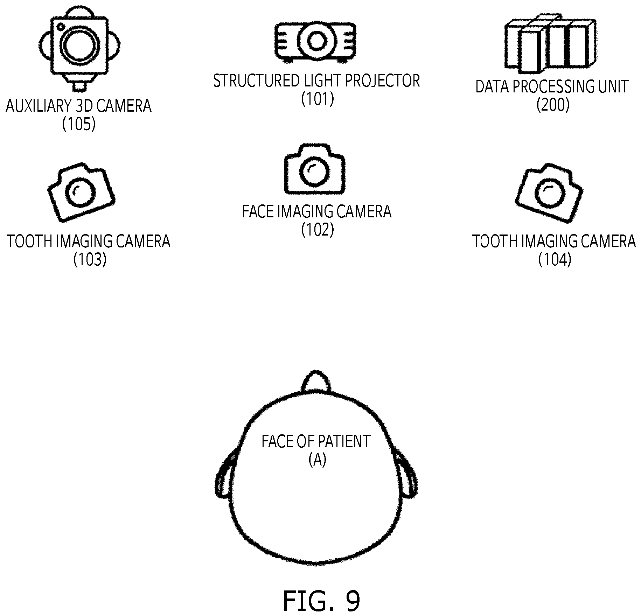

FIG. 9 illustrates a 3D face scan system, and FIG. 10 is a flowchart illustrating a method for generating 3D face scan data.

Compared to FIG. 1, the example shown in FIG. 9 is further provided with an auxiliary 3D camera 105. If the face imaging camera 102 is used to photograph the front of the face, the auxiliary 3D camera 105 is configured to acquire 3D data on areas other than the front of the face, such as the side of the face or the chin area. It is preferable that the subject for photography of the auxiliary 3D camera 105 may be set by the operation of an administrator (e.g., by a dentist).

Accordingly, the example illustrated in FIG. 10 is different from the example shown in FIG. 2 in that auxiliary area 3D data for a preset area or a plurality of preset areas in the face of the patient (e.g., side face and chin area) is generated by the auxiliary 3D camera 105 in S250 and that the process of aligning and inserting the tooth zone contour 3D data and the auxiliary area 3D data into the frontal face 3D data is performed in S280.

On the other hand, the present disclosure may be implemented in the form of computer-readable code in a computer-readable non-transitory recording medium such as a non-volatile recording medium. The non-volatile recording medium includes various forms of storage devices such as hard disks, SSDs, CDROMs, NAS, magnetic tapes, web disks, cloud disks, etc., and may be implemented in the form in which codes are distributed and stored and executed in multiple storage devices connected by a network. In addition, the aspects of the present disclosure may be implemented in the form of a computer program stored in a medium for executing a specific procedure in conjunction with hardware.

## Claims

1. A method for generating three-dimensional (3D) face scan data, the method comprising:
acquiring a face image obtained by photographing a face of a patient;
generating face 3D data from the face image; acquiring a tooth area image obtained by photographing a tooth area of the patient;
generating tooth area 3D data from the tooth area image;
extracting a tooth zone contour for the face image or the face 3D data;
separating and acquiring tooth zone contour 3D data corresponding to the tooth zone contour from the tooth area 3D data; and
aligning and inserting the tooth zone contour 3D data into the face 3D data.

2. The method according to claim 1, wherein
the acquiring the tooth area image obtained by photographing the tooth area of the patient includes acquiring a plurality of partial tooth area images obtained by photographing the tooth area of the patient from a plurality of angles, and
wherein the generating the tooth area 3D data from the tooth area image includes: generating a plurality of partial tooth area 3D data from the plurality of partial tooth area images; and generating the tooth area 3D data for the whole tooth area by aligning the plurality of partial tooth area 3D data.

3. The method according to claim 2, wherein the aligning and inserting the tooth zone contour 3D data into the face 3D data includes:
removing data of the tooth zone contour portion from the face 3D data; and
aligning and inserting the tooth zone contour 3D data into the tooth zone contour portion of the face 3D data.

4. The method according to claim 3, the method further comprising:
generating auxiliary area 3D data for a preset area of a face; and
aligning and inserting the auxiliary area 3D data into the face 3D data.

5. The method according to claim 1, wherein the face image, the face 3D data, the tooth area image, and the tooth area 3D data are acquired while a structured light pattern is projected onto the face or the tooth area of the patient.

6. The method according to claim 1, wherein the extracting the tooth zone contour for the face image or the face 3D data includes:
recognizing face landmarks from the face image or the face 3D data; and
extracting a tooth zone contour for the face image or the face 3D data on the basis of the face landmarks.

7. A computer program stored in a storage medium to execute the method of generating 3D face scan data according to any one of claims 1 to 6 by a computer.
